# EUROPÄISCHE PATENTSCHRIFT

(11) **EP 1 771 211 B1**
(45) Veröffentlichungstag und Bekanntmachung des Hinweises auf die Patenterteilung: **04.01.2012**
(21) Anmeldenummer: 05760685.7
(22) Anmeldetag: 16.07.2005
(51) Int. Cl.: A61L 2/14, B05D 3/06, B65B 55/08, H01H 37/32

(54) **VORRICHTUNG ZUR PLASMABESCHICHTUNG/STERILISATION**
DEVICE FOR PLASMA COATING/STERILIZATION
DISPOSITIF DE REVETEMENT PLASMA/STERILISATION

(30) Priorität: 24.07.2004 DE 102004036063
(43) Veröffentlichungstag der Anmeldung: 11.04.2007
(73) Patentinhaber: Krones AG, 93073 Neutraubling (DE)
(72) Erfinder: ZIMMERER, Johann, 93170 Bernhardswald (DE); HUMELE, Heinz, 93107 Thalmassing (DE)
(74) Vertreter: Grünecker, Kinkeldey, Stockmair & Schwanhäusser
(86) Internationale Anmeldenummer: PCT/EP2005/007773
(87) Internationale Veröffentlichungsnummer: WO 2006/010509

(56) Entgegenhaltungen:
- CH-A- 502 774
- DE-A1- 4 407 183
- DE-A1- 10 236 683
- DE-A1- 19 517 998
- DE-A1- 19 909 826
- DE-B- 1 204 587
- US-A- 3 421 229
- US-A- 6 051 296
- US-A- 6 057 004
- US-A1- 2002 006 487
- US-B1- 6 342 187
- PATENT ABSTRACTS OF JAPAN Bd. 017, Nr. 274 (C-1064), 27. Mai 1993 (1993-05-27) & JP 05 009317 A (MITSUBISHI PETROCHEM CO LTD), 19. Januar 1993 (1993-01-19)

## Beschreibung

Die Erfindung bezieht sich auf eine Vorrichtung zum Behandeln von Behältern.

Eine derartige Vorrichtung ist aus der DE-A-102 36 683 bekannt.

Diese Druckschrift zeigt eine Vorrichtung zur Plasmabehandlung von Hohlkörpern, insbesondere von Flaschen, in Ausführungsbeispielen, die eine Ausgestaltung der Vorrichtung zum Sterilisieren des Innenraums von PET-Flaschen zeigen. Die Vorrichtung enthält einen Rotor, der sich innerhalb einer radial außenliegenden, feststehenden Wand um eine Achse dreht. Am Umfang des Rotors sind einzelne Kammern ausgebildet, die jeweils einen Behälter aufweisen. Die Kammer mit dem Behälter fährt an der stationären äußeren Wand entlang, wobei die Kammer abdichtend an der äußeren Wand anliegt. Die Kammer hat einen bewegbaren Boden, auf den im Bereich einer Schleuse der Behälter gesetzt wird. Dies geschieht mit Hilfe eines Greifers, der an seinem Umfang mit Greifzangen versehen ist, die mit dem Einlaufstern (der Schleuse) rotieren. Die Umlaufwege der Kammern am Rotor und der Greifzangen am Einlaufstern überlappen sich, so dass der Behälter durch Öffnen der Greifzange des Einlaufsterns im entsprechenden Moment auf den Boden abgestellt wird. Nach dem Öffnen der Greifzange fährt der Boden nach oben und bringt den Behälter in die Kammer des Rotors hinein. Gleichzeitig dichtet der Boden die Kammern nach unten hin ab. Während der Bewegung des Rotors verbleibt der Behälter stationär in der Behandlungskammer. Es wird erwähnt, dass die Erfindung, d.h. die Abdichtung der Kammer durch eine stationäre Wand, auch zum Sterilisieren und/oder Beschichten eingesetzt werden kann. Wie die Beschichtung jedoch stattfinden soll, ist dieser Druckschrift nicht zu entnehmen.

Bekannt ist aus der DE 698 15 359 T2 ein Verfahren bei dem Plastikbehälter auf ihrer Außenseite mit einer Gassperrenbeschichtung versehen werden können. Hierzu wird ein Beschichtungsmaterial verdampft und die Plastikbehälter in verschiedenen Orientierungen über die Verdampfungsquelle geführt.

Nachteilig ist hierbei ein hoher mechanischer Aufwand sowie eine ungenaue Verteilung der Beschichtung auf den unregelmäßig geformten Flaschenkörpern.

Derartige Gassperrenschichten dienen dazu, die Verflüchtigung von beispielsweise CO₂ aus Getränken zu verzögern, um so die Mindesthaltbarkeitsdauer zu erhöhen.

Aus der DE 101 34 037 ist eine Vorrichtung zur Plasmasterilisation von Behältern, insbesondere von PET-Flaschen in einer Plasmakammer bekannt. Hier werden PET-Flaschen in eine Plasmakammer ein- und ausgeschleust und mittels eines Plasmas sterilisiert. Dies kann beispielsweise unmittelbar vor dem Befüllen der Flaschen erfolgen, um das Füllgut keimfrei zu halten.

Aus der EP 1 009 710 B1 ist ferner eine Vorrichtung zum Einschleusen von Behältern in einen Behandlungsraum bekannt.

Aufgabe der vorliegenden Erfindung ist es, die bekannten Vorrichtungen zum Behandeln von Behältern zu verbessern. Diese Aufgabe wird mit einer Vorrichtung nach Anspruch 1, gelöst.

Vorteilhafte Ausführungsformen sind in den Unteransprüchen offenbart.

Die Vorrichtung ist so ausgebildet sind, dass die Behälter sowohl sterilisiert als auch beschichtet werden. Dabei ist die Reihenfolge der Bearbeitungsvorgänge sterilisieren und beschichten beliebig zu wählen. Es ist auch denkbar, dass die Sterilisation gleichzeitig mit der Beschichtung stattfindet bzw. dass die Beschichtungsparameter so gewählt sind, dass auch gleichzeitig sterilisiert wird. Finden die Prozesse getrennt voneinander statt, so ist es trotzdem denkbar, dass sie in ein und derselben Behandlungskammer durchgeführt werden. Die Beschichtung erfolgt vorzugsweise auf der Innenseite der Behälter.

Es ist ein Plasmabehandlungsbereich vorhanden, der ringförmig bzw. ringsegmentförmig ist, durch die die Behälter geführt werden. Dies erlaubt eine einfache Konstruktion des Behältertransports, da diese dann beispielsweise an einem Rotor hängend durch den ringsegmentförmigen Plasmabehandlungsbereich geführt werden können und ist Leistungserhöhend im Gegensatz zu einem Prozess, der Einzel- bzw. Doppelkammern zur Behandlung der Behälter verwendet.

Da hier recht viele Halter oder Greifer für die Behälter nötig sind, sind die Greifen als Doppelgreifer ausgebildet die gleichzeitig zwei Behälter fassen Können. Damit ist es nicht nötig, für jeden einzelnen Behälter einen Greifer vorzusehen, der über verschiedene mechanische Verstellmechanismen verfügen muss. Dabei werden Schleusen verwendet, die über besonders ausgebildete Zellen verfügen. Diese Zellen können mehrere Flaschen gleichzeitig aufnehmen und erlauben so bei einer vereinfachten Konstruktion einen höheren Flaschendurchsatz.

Weiterhin ist der Boden der Behandlungskammer insoweit angepasst, dass der Innenraum des Plasmabehandlungsbereichs möglichst klein gehalten wird. Dies ist zur Erzeugung von beispielsweise einem Vakuum in der Behandlungskammer von Vorteil.

Bevorzugt ist weiterhin eine Ausführungsform, bei der die Behälter in ihrer Höhe variiert werden können. Dadurch ist es möglich, die Behälter in eine Behandlungsposition zu bringen. Insbesondere, falls in dem Behälter eine Elektrode, ein Mikrowellenleiter oder ein Rohr zur Zuführung eines Prozessgases angeordnet werden soll, kann der Behälter mit der oben liegenden Öffnung von unten nach oben soweit verschoben werden, dass sich die Elektrode, der Mikrowellenleiter oder das Rohr zumindest teilweise innerhalb des Behälters befindet. Entsprechendes ist auch bei einer Horizontalbewegung einer liegenden Flasche möglich oder bei einer Anordnung bei der die Flasche mit der Öffnung nach unten zeigt, sodass sie mit einer Bewegung nach unten über das Material oder über die Elektrode gestülpt wird.

Besonders vorteilhaft ist eine Ausführungsform, bei der in der Behandlungskammer ein Hohlkörper vorgesehen ist, der sich mit den Behältern mitbewegt. Dies erlaubt Durchführungen von Atmosphärendruck zu einem Niedrigdruck in der Behandlungskammer durch eine mitrotierende wand hindurch. Der Übergang von einer feststehenden zu einer rotierenden Versorgungsleitung kann dann unter normalem Atmosphärendruckbedingungen erfolgen.

In einer vorteilhaften Ausführungsform sind die Schleusen dazu da, um die zu behandelnden Behälter von Atmosphärendruck in einen Unterdruckbehandlungsraum zu überführen. Im Verlauf der Bewegung der Behälter durch die Schleuse wird der Unterdruck mit Hilfe von Pumpen erzeugt. Dabei können sowohl mehrere Pumpen am Umfang der Schleuse vorhanden sein, als auch eine zentrale Pumpe, die den Unterdruck mittels Anschlüssen am Umfang der Schleuse erzeugt.

Vorteilhaft sind weiterhin UV-Lampen, zur Behandlung der Behälter. Diese UV-Lampen können beispielsweise bei der Sterilisation hilfreich sein.

Ausführungsformen der Vorrichtung des Verfahrens werden anhand der beiliegenden Figuren erläutert. Dabei zeigt:
- Figur 1: eine schematische Draufsicht auf eine Vorrichtung zum Behandeln von Behältern;
- Figur 2: eine schematische Draufsicht auf eine Schleuse;
- Figur 3: eine schematische Draufsicht auf die Behandlungskammer;
- Figur 4: eine dreidimensionale schematische Ansicht der Vorrichtung zum Behandeln von Behältern;
- Figur 5: eine schematische Schnittansicht der Vorrichtung zum Behandeln von Behältern;
- Figur 6: eine schematische dreidimensionale Darstellung eines Doppelgreifers.

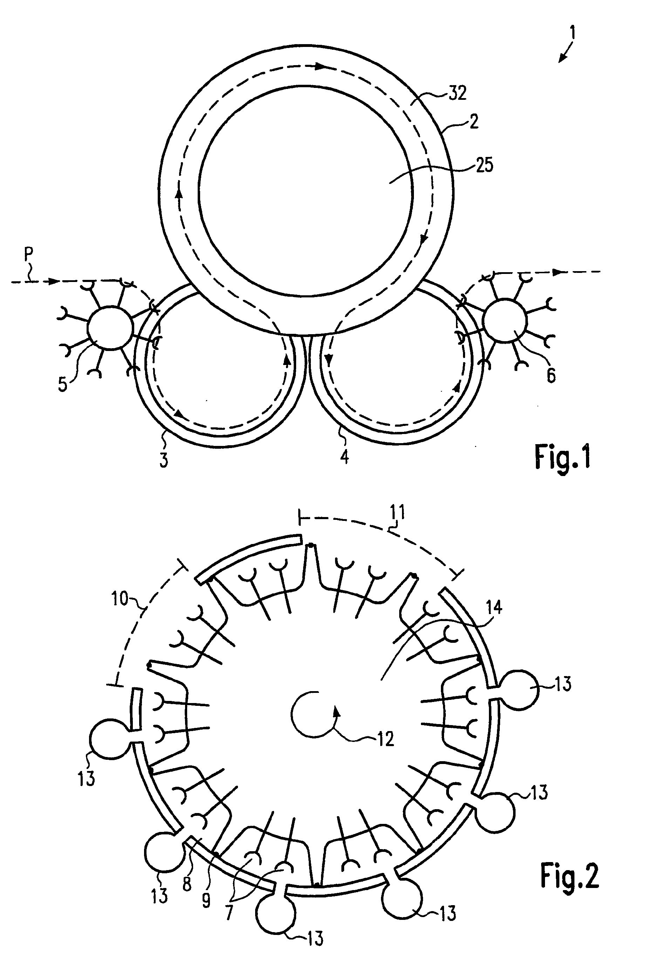

In Figur 1 ist in Draufsicht eine Vorrichtung 1 zum Behandeln von Behältern gezeigt. Der vorgesehene Weg der Behälter ist durch die gestrichelte Linie P dargestellt. Von einem (nicht dargestellten) Zuförderer werden die Behälter mit einem Transferstern 5 an eine Eingangsschleusenkammer 3 übergeben. Von dort können die Behälter in die Behandlungskammer 2 gelangen, um nach einem Umlauf dort mit der Ausgangsschleuse 4 ausgeschleust und mit einem Transferstern 4 auf einen (nicht dargestellten) Abförderer transferiert zu werden.

Die Behandlungskammer 2 verfügt über einen Plasmabehandlungsbereich 32, in dem die Behälter umlaufen können. In der Behandlungskammer 2 ist ein Hohlkörper 25 angeordnet. Dadurch ergibt sich ein ringförmiger Plasmabehandlungsbereich 32. Der Hohlkörper 25 verringert vorteilhafterweise das zu evakuierende Volumen des Plasmabehandlungsbereichs 32. Je größer der Hohlkörper desto geringer ist der Platz des Plasmabehandlungsbereichs 32. Die Schleusen 3 und 4 sowie die Behandlungskammer 2 verfügen über rotierende Transportmittel, mit denen die Behälter auf Kreissegmentabschnitten transportiert werden können und mit denen sie zwischen den Schleusen 3, 4 und der Behandlungskammer 2 übergeben werden können.

In Figur 2 ist eine detailliertere Draufsicht auf die Schleuse 3 dargestellt. Die Schleuse 4 ist im Prinzip ähnlich aufgebaut, nur dass bei ihr die Behälter nicht evakuiert, sondern geflutet werden. In einem Bereich 10, der in Figur 2 gestrichelt dargestellt ist, können Behälter in die Schleuse 3 eingesetzt werden. Zur Aufnahme der Behälter sind Greifer 7 schematisch dargestellt. Die Greifer 7 können so ausgestaltet sein, dass sie von außen betätigt werden, um die Flaschen aufzunehmen oder abzugeben. Während des Umlaufs zwischen der Eingabestelle 10 und der Ausgabestelle 11 sind die Greifer beispielsweise so arretiert, dass die Behälter fest gehalten sind.

Vorteilhaft ist hierbei, dass die Greifer 7 so ausgebildet sind, dass sie PET-Flaschen unterhalb ihres Kragens fassen.

In der Schleuse 3 ist ein Rotor 14 vorgesehen, der sich in Richtung 12 drehen kann. Das bedeutet, dass die Behälter von dem Eingabebereich 10 entlang dem längeren Umfang zu der Ausgabestelle 11 transportiert werden. Dadurch ergibt sich ein möglichst langer Weg in der Schleuse, um von Atmosphärendruck bei dem Eingangsbereich 10 auf einen Niederdruck bei dem Ausgangsbereich 11 zu kommen. Der zentrale Rotor verfügt über Zellen 8, die zur Aufnahme der Behälter dienen. Die verschiedenen Zellen 8 sind mit Dichtungen 9 vertikal und mit nicht gezeigten dynamischen Dichtungen horizontal gegeneinander abgedichtet, wobei die Dichtungen 9 den Rotor 14 gegen die Außenwand hin abdichten und die nicht gezeigten dynamischen Dichtungen den Rotor 14 gegen den Boden und die Decke hin abdichten.. Die in Figur 2 beispielsweise unten dargestellten Zellen sind so nach außen hin hermetisch abgeschlossen. Mittels Vakuumpumpen 13, die entlang der Schleusenaußenseite verteilt sind, können die Zellen 8 abgepumpt werden.

Dabei wird auf dem Weg vom Eingangsbereich 10 zum Ausgangsbereich 11 in Umlaufrichtung 12 der Druck in den Zellen 8 mit fortschreitender Evakuierung immer niedriger. Die Zellen 8 sind so ausgebildet, dass sie zwei Behälter aufnehmen können. Entsprechend sind zwei Greifer 7 pro Zelle 8 vorgesehen. Dies erlaubt eine dichtere Staffelung der Behälter in der Schleuse 3. Dadurch wird bei gleichbleibender Drehzahl ein höherer Durchsatz bei geringerem Konstruktionsaufwand für die Zellen erreicht.

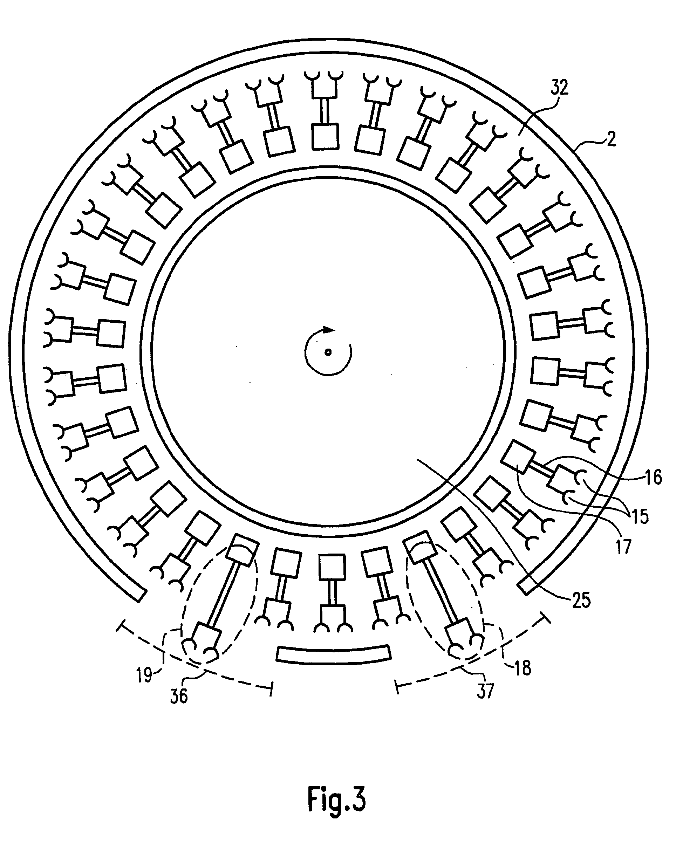

In Figur 3 ist eine Draufsicht auf die Behandlungskammer 2 gezeigt. Die Behandlungskammer 2 verfügt über eine Mehrzahl von Doppelgreifern. Jeder Doppelgreifer verfügt über zwei Greifelemente 15, von denen jedes einen Behälter greifen kann. Die Greifelemente 15 sind an einer Haltestange 16 angeordnet, die ihrerseits an einer Führung 17 gelagert ist. Die Doppelgreifer sind entlang eines Rotors angeordnet, der in der Behandlungskammer 2 umlaufen kann.

In einem Bereich 36 können die Behälter an die Greifer der Behandlungskammer 2 übergeben werden. Dazu kann (wie bei Bezugsziffer 19 dargestellt) ein Doppelgreifer so ausgefahren werden, dass er bis in die in Figur 2 gezeigte Schleusenkammer reicht, um dort ankommende Behälter aufnehmen zu können. Entsprechend können die Behälter nach dem Umlauf in der Behandlungskammer 2 (wie bei Bezugsziffer 18 gezeigt) mit dem Doppelgreifer radial herausbewegt werden, um so von den Greifern einer Ausgangsschleuse aufgenommen zu werden. In dem Raum innerhalb der ringförmig angeordneten Doppelgreifer ist der zentrale Hohlkörper 25 angeordnet. Dieser Hohlkörper kann der Rotor sein, an dem die Doppelgreifer befestigt sind.

Die Greifer 15 sind so angeordnet, dass die Abstände der gehaltenen Behälter zu den benachbarten Behältern immer gleich ist. Dadurch wird eine gleichmäßige Behandlung aller Behälter sichergestellt.

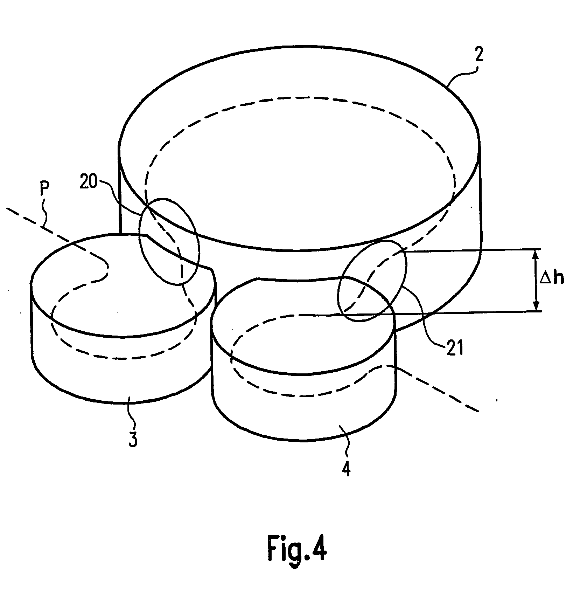

In Figur 4 ist eine schematische Darstellung der Vorrichtung zur Behandlung der Behälter in dreidimensionaler Ansicht dargestellt. Der Pfad P der Behälter ist wie in Figur 1 gestrichelt dargestellt.

Wie in Figur 4 zu erkennen ist, liegen die Schleusen 3, 4 niedriger als die Behandlungskammer 2. Dadurch können die Behälter auf einem relativ niedrigen Höhenniveau in die Behandlungskammer 2 eingeführt werden, um dort im Bereich 20 angehoben zu werden. Im Bereich 21 können die Behälter auf ihrer Bahn abgesenkt werden, um so an die Schleuse 4 abgegeben werden zu können. Durch das Anheben der Behälter im Bereich 20 können die Behälter innerhalb der Behandlungskammer 2 in ihre Behandlungsposition gebracht werden. Eben können sie so im Bereich 21 aus der Behandlungsposition herausgebracht werden, damit die Übergabe an die Schleusenkammer 4 erfolgen kann.

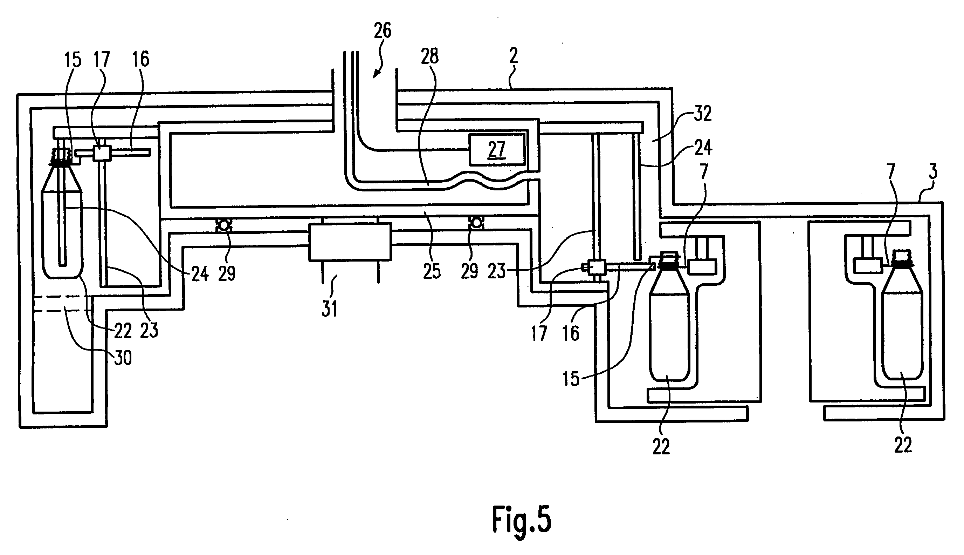

In Figur 5 ist eine schematische Schnittzeichnung der Behandlungskammer 2 und der Schleus 3 dargestellt.

In der Schleuse 3 ist ein Greifer 7 dargestellt, der einen Behälter 22 hält. Wie zu sehen ist, wird der Behälter 22 unterhalb eines Tragrandes gehalten. Dies ermöglicht, dass der Greifer 15 der Behandlungskammer 2 den Behälter oberhalb des Tragrandes fasst, sodass der gesamte Behälter unterhalb des Tragrandes frei zugänglich ist, was für das Beschichten von Vorteil sein kann.

Wie in Figur 5 zu erkennen ist, sind die Greifer 15 an einer verdrehfesten Stange 16 befestigt, die in Figur 5 in einem nach außen hin ausgefahrenen Zustand dargestellt ist, sodass der Greifer 15 den Behälter 22 in der Schleuse 3 greifen kann. Die Stange 16 ist an der Lagerung 17 horizontal beweglich ausgeführt, sodass durch Zurückziehen der Stange 16 zum Zentrum der Behandlungskammer 2 hin der Behälter 22 in den Bereich der Behandlungskammer 2 gelangt. Anschließend kann er durch Verschieben der Lagerung 17 entlang der Stange 23 nach oben hin bewegt werden, sodass die Elektrode 24 bzw. der Mikrowellenleiter 24 bzw. das Rohr 24 in das Innere des Behälters 22 ragt. Hier und im Folgenden wird ein Mikrowellenleiter stellvertretend für beliebige Beschichtungsvorrichtungen aufgeführt. In dieser angehobenen Position ist der Behälter 22 in seiner Behandlungsposition. Die Halterung des Behälters 22 in der Behandlungskammer 2 ist insgesamt an dem Hohlkörper 25 befestigt. Der Hohlkörper 25 ist rotierbar gelagert (siehe Lagerung 29), sodass die Greifer mit den Behältern 22 zusammen mit dem Hohlkörper 25 in der Behandlungskammer 2 umlaufen können.

In Figur 5 ist links ein Behälter 22 in der Behandlungsposition dargestellt. Die Elektrode bzw. der Mikrowellenleiter 24 ist im Inneren des Behälters 22 angeordnet. Die Lagerung 17 mit der Stange 16 und den Greifern 15 ist in einer angehobenen Position dargestellt.

Der Hohlkörper 25 ist über Zugänge 26, 31 mit der Umgebung verbunden. Durch den Zugang 26 können Versorgungsleitungen wie etwa eine Spannungsversorgung für einen Mikrowellengenerator 27 oder eine Gaszufuhr 28 geführt werden. Über den Zugang 31 können Versorgungsleitungen wie etwa für Kühlfluida geführt werden. Der Zugang erfolgt dabei z.B. mittels magnetfluidgedichteter Drehdurchführungen. Der Hohlkörper 25 kann über eine Antriebswelle 31 angetrieben werden. Sowohl unterhalb des Hohlkörpers 25 (etwa bei den Lagern 29) als auch oberhalb des Hohlkörpers 25 kann Vakuum vorgesehen sein, das durch die äußere Wandung der Behandlungskammer 2, wie dargestellt, begrenzt wird.

Ein Gaseinlass 28, wie er in Figur 5 dargestellt ist, kann jedoch auch an dem feststehenden Teil der Behandlungskammer 2 angeordnet sein, sodass die Einleitung des Prozessgases erleichtert wird.

Vorteilhaft ist es insbesondere, wenn mehrere Einlässe für verschiedene Fluida zur verfügung stehen, um so ein geeignetes Fluidgemisch zu erhalten.

Zum Erzeugen des Plasmas können Mikrowellen- oder Hochfrequenzgeneratoren eingesetzt werden. Entsprechende Elektroden, Wellenleiter, Magnete oder ähnliches sind in den Figuren der Übersichtlichkeit halber nur schematisch oder gar nicht dargestellt.

In Figur 5 ist links ein Boden 30 eingezeichnet. Unterhalb des Bodens 30 ist der Innenraum, der auf der rechten Seite für das Ein- und Ausschleusen der Behälter 22 benötigt wird. Auf der linken Seite (hier findet die Behandlung der Behälter 22 statt) ist dieser untere Raum jedoch nicht nötig, sodass er durch einen Boden 30 abgeschlossen wird. Auch kann dann das Volumen unterhalb des Bodens 30 wegfallen.

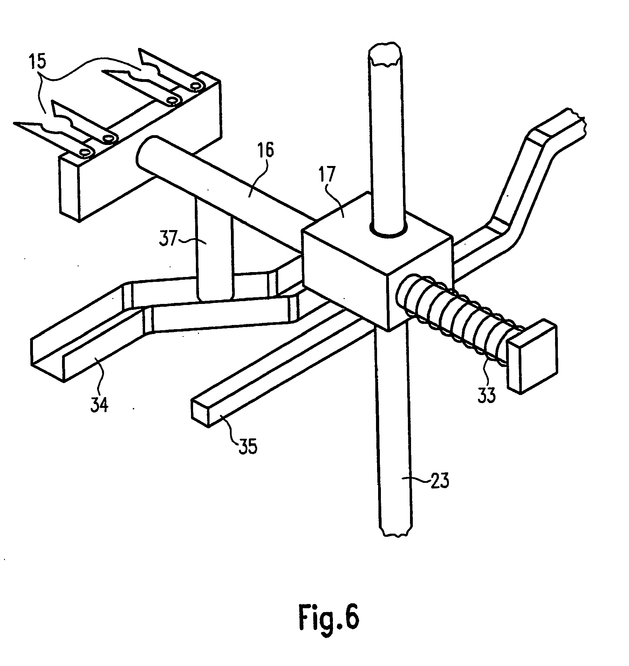

In Figur 6 sind die Doppelgreifer detailliert dargestellt. An einer Stange 23 ist höhenbeweglich ein Lager 17 angeordnet. In dem Lager 17 ist horizontal beweglich eine Stange 16 vorgesehen. Am Ende der Stange 16 sind über eine Platte zwei Greifer 15 vorgesehen, sodass jeweils für zwei Greifer nur einmal die Mechanik bestehend aus Stange 16, Lager 17 und Stange 23, die jeweils verdrehsicher gelagert sind, notwendig ist. Die beiden Stangen 16 und 23 sind mit einer Gleitbeschichtung aus Wolfram-Disulfid versehen, die einen wartungsfreien Einsatz in der Behandlungskammer ermöglicht. Durch diese Art von Doppelgreifern sind die mechanischen Aufwendungen im Vergleich zu Einzelgreifern verringert. Etwaige Mechanik oder Betätigungsmittel zum Öffnen und Schließen der Greifer 15, wie etwa Steuerkurven, sind in Fig. 6 der Übersichtlichkeit halber nicht dargestellt. Auch sind die Greifer 15 vereinfacht dargestellt.

Zur Horizontalverstellung der Greifer 15 ist eine Kurvenbahn 34 vorgesehen, in die ein Führungsglied 37 der Stange 16 ragt. In Figur 6 ist die Steuerkurve 34 so vorgesehen, dass sie die Stange 16 sowohl nach rechts als auch nach links verschieben kann. Jedoch ist es auch möglich, die Stange 16 in eine der beiden Richtungen durch eine Feder 33 vorzuspannen, sodass die Steuerkurve 34 lediglich so ausgebildet sein muss, dass sie die Bewegung der Stange 16 entgegen der Federkraft der Feder 33 bewirkt. Eine zweite Steuerkurve 35 ist vorgesehen, um die vertikale Position des Lagers 17 an der Stange 23 einzustellen. Hierbei wird das Lager 17 durch Kontakt mit der Steuerkurve 35 in seiner Höhenposition fixiert. Eine Kraft für eine Bewegung nach unten wird entweder durch die Schwerkraft oder durch eine zusätzliche Feder oder durch eine weitere Steuerkurve bewirkt.

Prinzipiell ist es auch möglich, eine einzelne Steuerkurve vorzusehen, die die Stange 16 in der horizontalen und das Lager 17 und in der vertikalen Richtung führt. Würde z. B. die Steuerkurve 34 nach oben gezogen, so wie dies in Figur 6 rechts hinten für die Steuerkurve 35 dargestellt ist, so würde die Stange 16 zusammen mit dem Lager 17 angehoben.

Zur Durchführung eines Verfahrens unter Verwendung der erfindungsgemäßen vorrichtung werden Behälter 22 über Transfersterne 5 in die Greifer 7 der Schleuse 3 transferiert. Da die Greifer 7 nicht äquidistant entlang des Umfangs der Schleuse 3 angeordnet sind, muss der Transferstern 5 entsprechend ausgestaltet sein, die Behälter 22 abwechselnd mit großem und kleinem Zwischenabstand an die Greifer 7 der Schleuse 3 übergeben zu können. Die in einer Zelle 8 der Schleuse 3 angeordneten Flaschen rotieren im Drehsinn 12 von dem Eingangsbereich 10 zum Ausgangsbereich 11, währenddessen sie evakuiert werden. Dazu wird das Innere einer Zelle 8 mittels der Pumpen 13 evakuiert. Der Druck in den Zellen 8 ist im Bereich der Ausgabestelle 11 unterhalb von einem Millibar, wie beispielsweise bei etwa 0,1 Millibar.

Im Bereich der Stelle 11 (entsprechend Bezugszeichen 36 in Figur 3) fahren die Doppelgreifer der Behandlungskammer 2 (siehe Bezugsziffer 19) aus und übernehmen Behälter 22 aus einer Zelle 8 der Schleuse 3. Daraufhin fahren die Doppelgreifer in Richtung des Zentrums der Behandlungskammer 2 zurück und anschließend nach oben, sodass die Behälter 22 über die Elektrode 24 bzw. den Mikrowellenleiter 24 bzw. das Rohr 24 geführt werden. Anschließend werden die Behälter 22 durch die Behandlungskammer 2 im Uhrzeigersinn geführt, wobei sie durch Plasmabereiche hindurchtreten. In diesen Plasmabereichen wird die Innen- und / oder Außenseite der Behälter 22 sterilisiert und/oder mit einer Beschichtung versehen. Die Beschichtung der Behälter 22 dient dazu, die Gasdurchlässigkeit beispielsweise für CO₂ zu verringern, umso die Mindesthaltbarkeit CO₂-haltiger Getränke zu verlängern.

Als Plasma kommt ein Niederdruckplasma (ca. 0,1 Pa) in Betracht, da es zum Einen "kalt" ist und so beispielsweise PET-Behälter nicht beschädigt. Als Prozessgase können beispielsweise Argon, Sauerstoff, Kohlendioxid, Wasserstoff, Stickstoff, Ammoniak oder Luft eingesetzt werden.

Zum Beschichten der Behälter 22 an ihrer Innenseite wird reaktives Material im Inneren der Behälter 22 angeordnet. Dies kann beispielsweise mit den Elektroden- oder Rohren 24 geschehen. Quarzglasstäbe 24 resultieren in einer SiO₂-Beschichtung der Innenseite der Behälter 22, die optisch transparent und für Lebensmittel unbedenklich ist.

Vorteilhaft ist es weiterhin, TiO₂ aufzubringen, das vorteilhafterweise als Nanoteilchen in einer SiO₂-Matrix eingebunden ist.

TiO₂ ist fotochemisch aktiv. Dies bedeutet, bei Beleuchtung mit UV-Strahlung, die vorzugsweise von der Außenseite des Behälters angebracht wird, (beispielsweise zwischen 200 und 400 nm Wellenlänge) weist TiO₂ ein hohes Oxidationspotenzial auf und vermag organische Molekühle (d. h. z.B. Keime) zu oxidieren, sodass eine Sterilisation erfolgt.

Durch geeignete Wahl der Prozessgase bzw. von geeignetem Beschichtungsmaterial ist es somit möglich, gleichzeitig oder nacheinander in der Behandlungskammer 2 sowohl ein Beschichten als auch ein Sterilisieren von Behältern an ihrer Innen- und / oder Außenseite vorzusehen.

Nach oder während dem Aufbringen einer SiO₂-Schicht (vorzugsweise mit TiO₂ in Form von Nanoteilchen; wobei das TiO₂ bevorzugterweise in der Kristallmodifikation Anatas vorliegt) kann durch Beleuchten mit UV-Licht die Sterilisation erfolgen. Das UV-Licht kann aus dem Plasma selbst stammen oder auch durch UV-Lampen erzeugt werden. Die UV-Lampen können hier auch beispielsweise im Bereich der Ausgangsschleuse angeordnet sein, um so auch die Zeit des Ausschleusens zum Sterilisieren nutzen zu können. Auch können die UV-Lampen im Bereich des Absenkens der Flaschen (siehe Bezugsziffer 21 in Figur 4) die Behälter 22 beleuchten.

Auch ist es möglich, dass beispielsweise in einer ersten Hälfte der Behandlungskammer 2 ein Plasma gezündet wird, das eine Beschichtung der Behälter 22 vornimmt und in der zweiten Hälfte ein Plasma gezündet wird, das die Sterilisation der Behälter vornimmt. Auch ist die umgekehrte Vorgehensweise möglich.

Vorteilhaft ist es jedoch, wenn das Beschichten und das Sterilisieren gleichzeitig stattfinden, da dann für beide Prozesse genügend Zeit zur Verfügung steht. Hierbei ist zu berücksichtigen, dass bei einem Durchmesser der Behandlungskammer 2 von etwa 2 bis 3 m und einem gewünschten Flaschendurchsatz von 20.000 bis 30.000 Flaschen pro Stunde die Verweilzeit der Behälter 22 in der Behandlungskammer 2 bei wenigen Sekunden, d. h. in der Größenordnung von weniger als 10 Sekunden wie beispielsweise nur fünf Sekunden, liegt. In dieser recht kurzen zeit muss eine ausreichende Sterilisation/Beschichtung der Behälter gewährleistet werden.

Nach der Ausgabe der Behälter 22 aus der Schleuse 4, können diese vorteilhafterweise über einen sterilen Förderer weiterbefördert werden wie etwa zu einem Füller.

Die verschiedenen maschinenbaulichen Aspekte, wie etwa die Höhe der Schleusen 3, 4 im Vergleich zu der Behandlungskammer 2, die Zellen 8 für mindestens zwei Behälter 22, der Hohlkörper 25 im Vakuum, die Höhenveränderung der Behälter um sie in die Behandlungsposition zu bringen, der ringsegmentförmige Plasmabehandlungsbereich 32, die verschiedenen Bodenhöhen im Plasmabehandlungsbereich 32, die verschiedenen Steuerkurven 34, 35 für die Greifer, 15 etc. sind vorteilhaft unabhängig davon, ob das Verfahren bzw. die Behandlungskammer 2 zum Beschichten und Sterilisieren oder auch nur zum Beschichten oder Sterilisieren vorgesehen sind.

## Patentansprüche

1. Vorrichtung (1) zum Behandeln von Behältern (22), wie etwa Flaschen, vorzugsweise PET-Behältern wie etwa PET-Flaschen, mit einem Plasma zum Sterilisieren und zum Beschichten der Behälter (22), wobei die Vorrichtung eine Behandlungskammer (2) und eine oder zwei Schleusen (3, 4) zum Ein- und/oder Ausschleusen der Behälter (22) in und aus der Behandlungskammer (2) sowie einen zum Behältertransport während der Behandlung dienenden Rotor (25) umfasst und für den Transport der Behälter (22) in der Schleuse/den Schleusen (3, 4) und in der Behandlungskammer (2) zwei verschiedene Niveaus vorgesehen sind, wobei die Behandlungskammer (2) einen ringförmigen und/oder ringsegmentförmigen Plasmabehandlungsbereich (32) enthält, den die Behälter (22) durchlaufen, wobei der Boden (30) der Behandlungskammer (2) mindestens zwei verschiedene Höhenniveaus aufweist, derart, dass die Behälter (22) in den Schleusen (3,4) auf einem anderen Höhenniveau führbar sind, als in der Behandlungskammer (2) bei der Behandlung, wobei die Schleusenkammer/Schleusenkammern (3, 4) über Zellen (8) zur Aufnahme von mindestens, vorzugsweise genau, zwei Behältern (22) dient und der Rotor (25) über höhen- und horizontal verstellbare Doppelgreifer (15) zum gleichzeiteigen Greifen von zwei Behältern (22) verfügt.

2. Vorrichtung nach Anspruch 1, **dadurch gekennzeichnet, dass** die Behandlungskammer (2) eine Niederdruck-Plasmakammer umfasst.

3. Vorrichtung nach Anspruch 1 oder 2, **dadurch gekennzeichnet, dass** die Doppelgreifer (15) so angeordnet sind, dass die dazugehörigen Behälterpositionen den jeweils gleichen Winkelabstand zu benachbarten Behälterpositionen haben.

4. Vorrichtung nach einem der Ansprüche 1 bis 3, **dadurch gekennzeichnet, dass** zur Höhenverstellung der Greifer (15) eine Steuerkurve (35) vorgesehen ist.

5. Vorrichtung nach einem der Ansprüche 1 bis 4, **dadurch gekennzeichnet, dass** Elektroden (24), Mikrowellenleiter (24) und/oder Beschichtungsmaterialien (24) zur Plasmaerzeugung vorgesehen sind, wobei die Höhenverstellung vorzugsweise so ausgebildet ist, dass der Behälter (22) zwischen einer ersten und einer zweiten Positionen bewegt werden kann, wobei sich in der ersten Position die Elektrode (24) und/oder der Mikrowellenleiter (24) und/oder das Beschichtungsmaterial(24) außerhalb des Behälters (22) befindet, und sie sich in der zweiten Position zumindest teilweise innerhalb des Behälters (22) befindet.

6. Vorrichtung nach einem der Ansprüche 1 bis 5, **dadurch gekennzeichnet, dass** zur Horizontalverstellung der Greifer (15) eine Steuerkurve (34) vorgesehen ist.

7. Vorrichtung nach einem der Ansprüche 1 bis 6, **dadurch gekennzeichnet, dass** der Rotor (25) einen zentralen mitrotierenden Hohlkörper (25) umfasst, dessen Durchmesser bevorzugterweise mindestens 5 %, oder 10 % oder 20 % oder 30 % oder 40 % oder 50 % oder 75 oder 80 % oder 85 % oder 90 % des Durchmessers der Behandlungskammer (2) beträgt.

8. Vorrichtung nach Anspruch 7, **dadurch gekennzeichnet, dass** der Hohlkörper (25) mit der Atmosphäre durchgängig verbunden ist.

9. Vorrichtung nach einem der Ansprüche 7 oder 8, **dadurch gekennzeichnet, dass** Versorgungsleitungen (28) für das Innere der Behandlungskammer (2) durch die Wandung des Hohlkörpers (25) geführt werden, wobei die Versorgungsleitungen (28) beispielsweise Gaszu- oder -ableitungen oder Kühlwasserzu- oder -ableitungen oder Hochspannungsversorgungsleitungen oder Hochfrequenzversorgungsleitungen sind.

10. Vorrichtung nach Anspruch 1, **dadurch gekennzeichnet, dass** der Plasmabehandlungsbereich (32) nach außen durch eine feststehende Vorrichtungswand begrenzt wird, innerhalb derer sich ein mitrotierender Hohlkörper (25) befindet, der vorzugsweise in seinem Inneren zum Vorliegen von Atmosphärendruck vorgesehen ist und durch den vorzugsweise Versorgungsleitungen (28) geführt werden können.

11. Vorrichtung nach Anspruch 1, **dadurch gekennzeichnet, dass** im Bereich des Beginns und des Endes des Plasmabehandlungsbereichs (32) der Boden niedriger ist als einem anderen Teil des Plasmabehandlungsbereichs (32).

12. Vorrichtung nach einem der Ansprüche 2 bis 11, **dadurch gekennzeichnet, dass** die Schleusenkammern (3, 4) über Greifer (7) für Behälter (22) verfügen, die bevorzugterweise auf einem drehbaren Rotor angeordnet sind.

13. Vorrichtung nach einem der Ansprüche 1 bis 12, **dadurch gekennzeichnet, dass** Übergabesterne (5, 6) bei den Schleusenkammern (3, 4) vorgesehen sind, mit denen Behälter (22) zu den Schleusen (3) hin oder von den Schleusen (4) weg transportiert werden können.

14. Vorrichtung nach einem der Ansprüche 1 bis 13, **dadurch gekennzeichnet, dass** UV-Lampen vorgesehen sind, mit denen die Behälter im Bereich der Schleuse (4), mit der Behälter (22) aus der Behandlungskammer (2) ausgeschleust werden können, beleuchtet werden können.

15. Vorrichtung nach einem der Ansprüche 1 bis 14, **dadurch gekennzeichnet, dass** Vorrichtungen zum Erzeugen von mindestens zwei verschiedenen Plasmas vorgesehen sind.

16. Vorrichtung nach Anspruch 15, **dadurch gekennzeichnet, dass** mindestens zwei Gaseinlässe für zwei verschiedene Gase vorgesehen sind.

17. Vorrichtung nach einem der Ansprüche 15 oder 16, **dadurch gekennzeichnet, dass** zwei verschiedene Abschnitte für die Erzeugung von zwei verschiedenen Plasmas vorgesehen sind.

18. Vorrichtung nach einem der Ansprüche 1 bis 17, **dadurch gekennzeichnet, dass** UV-Lampen zur Beleuchtung der Behälter (22) im Bereich der Behandlungskammer (2) und/oder im Bereich der Übergabe der Behälter (22) aus der Behandlungskammer (2) heraus angeordnet sind.

19. Vorrichtung nach Anspruch 1 bis 18, **dadurch gekennzeichnet, dass** ein und dasselbe Plasma sowohl zum Beschichten als auch zum Sterilisieren gebildet werden kann.

20. Vorrichtung nach Anspruch 19, **gekennzeichnet durch** ihre Ausbildung für eine Beschichtung mit SiO₂ und/oder TiO₂. vorzugsweise in der Kristallmodifikation Anatas, wobei vorzugsweise TiO₂ in eine SiO₂ Matrix als Nanoteilchen eingebaut ist.

## Claims

1. Device (1) for treating containers (22), such as bottles, preferably PET containers such as PET bottles, with a plasma for sterilising and for coating the containers (22), wherein the device comprises a treatment chamber (2) and one or two air locks (3, 4) for feeding and/or discharging the containers (22) in and out of the treatment chamber (2) as well as a rotor (25) serving to transport the containers during the treatment, and two different levels are provided for transporting the containers (22) into the air lock/air locks (3, 4) and into the treatment chamber (2), the treatment chamber (2) containing a ring-shaped and/or ring-segment-shaped plasma treatment area (32), through which the containers (22) pass, whereby the bottom (30) of the treatment chamber (2) has at least two different height levels, such that the containers (22) can be guided into the air locks (3, 4) at a different height level than into the treatment chamber (2) during the treatment, wherein the air lock chamber/air lock chambers (3, 4) serve(s) by means of cells (8) to receive at least, preferably exactly, two containers (22) and the rotor (25) has height and horizontally adjustable double grippers (15) for simultaneously gripping two containers (22).

2. Device according to claim 1, **characterised in that** the treatment chamber (2) comprises a low pressure plasma chamber.

3. Device according to claims 1 or 2, **characterised in that** the double grippers (15) are arranged such that the associated container positions have in each case the same angular distance to adjacent container positions.

4. Device according to any one of claims 1 to 3, **characterised in that** a control cam (35) is provided for adjusting the height of the grippers (15).

5. Device according to any one of claims 1 to 4, **characterised in that** electrodes (24), microwave conductors (24) and/or coating materials (24) are provided for generating the plasma, wherein the height adjustment is preferably performed such that the container (22) can be moved between a first and a second position, wherein in the first position the electrode (24) and/or the microwave conductor (24) and/or the coating material (24) is outside the container (22), and is at least partially inside the container (22) in the second position.

6. Device according to any one of claims 1 to 5, **characterised in that** a control cam (34) is provided for horizontally adjusting the grippers (15).

7. Device according to any one of claims 1 to 6, **characterised in that** the rotor (25) comprises a central co-rotating hollow body (25), whose diameter is preferably at least 5%, or 10% or 20% or 30% or 40% or 50% or 75 or 80% or 85% or 90% of the diameter of the treatment chamber (2).

8. Device according to claim 7, **characterised in that** the hollow body (25) is continuously connected to the atmosphere.

9. Device according to any one of claims 7 or 8, **characterised in that** supply lines (28) for the interior of the treatment chamber (2) are guided through the wall of the hollow body (25), wherein the supply lines (28) are for example gas feed lines or gas discharge lines or cooling water feed lines or cooling water discharge lines or high voltage supply lines or high frequency supply lines.

10. Device according to claim 1, **characterised in that** the plasma treatment area (32) is bordered to the outside by a stationary device wall, within which there is a co-rotating hollow body (25), which is preferably provided in its interior for atmospheric pressure to be present and through which the supply lines (28) can preferably be guided.

11. Device according to claim 1, **characterised in that** in the area of the beginning and the end of the plasma treatment area (32) the bottom is lower than another part of the plasma treatment area (32).

12. Device according to any one of claims 2 to 11, **characterised in that** the air lock chambers (3, 4) have grippers (7) for containers (22), which are preferably arranged on a rotatable rotor.

13. Device according to any one of claims 1 to 12, **characterised in that** transfer-stars (5, 6) are provided at the air lock chambers (3, 4), with which containers (22) can be transported toward the air locks (3) or away from the air locks (4).

14. Device according to any one of claims 1 to 13, **characterised in that** UV lamps are provided, with which UV lamps the containers can be illuminated in the area of the air lock (4), with which air lock (4) containers (22) can be discharged from the treatment chamber (2).

15. Device according to any one of claims 1 to 14, **characterised in that** devices for generating at least two different plasmas are provided.

16. Device according to claim 15, **characterised in that** at least two gas inlets are provided for two different gases.

17. Device according to any one of claims 15 or 16, **characterised in that** two different sections are provided for generating two different plasmas.

18. Device according to any one of claims 1 to 17, **characterised in that** UV lamps are provided for illuminating the containers (22) in the area of the treatment chamber (2) and/or in the area of the transfer of the containers (22) from the treatment chamber (2).

19. Device according to claim 1 to 18, **characterised in that** one and the same plasma may be used for both coating and sterilising.

20. Device according to claim 19, **characterised by** its design for a coating of SiO₂ and/or TiO₂, preferably in the anatase crystal modification, wherein TiO₂ is preferably incorporated into a SiO₂ matrix as nanoparticles.

## Revendications

1. Dispositif (1) pour traiter des récipients (22) tels que des bouteilles, de préférence des récipients en PET tels que des bouteilles en PET, avec un plasma en vue de les stériliser et de les revêtir, étant précisé que le dispositif comprend une chambre de traitement (2) et un ou deux sas (3, 4) pour amener les récipients (22) dans la chambre de traitement (2) et/ou les faire sortir de celle-ci, ainsi qu'un rotor (25) qui sert au transport des récipients pendant le traitement, et qu'il est prévu pour le transport des récipients (22) dans le/les sas (3, 4) et dans la chambre de traitement (2) deux niveaux différents, étant précisé que la chambre de traitement (2) contient une zone de traitement par plasma (32) annulaire et/ou en forme de segment d'anneau que les récipients (22) traversent, étant précisé que le fond (30) de la chambre de traitement (2) présente au moins deux niveaux de hauteur différents, de telle sorte que les récipients (22) qui se trouvent dans les sas (3, 4) peuvent être amenés à un niveau de hauteur différent de celui de la chambre de traitement (2) lors du traitement, et étant précisé que la/les chambres formant sas (3, 4) servent, par l'intermédiaire d'alvéoles (8), à recevoir au moins et de préférence exactement deux récipients (22) et que le rotor (25) dispose de grappins doubles (15), réglables en hauteur et horizontalement, pour saisir deux récipients (22) en même temps.

2. Dispositif selon la revendication 1, **caractérisé en ce que** la chambre de traitement (2) comprend une chambre de plasma basse pression.

3. Dispositif selon la revendication 1 ou 2, **caractérisé en ce que** les grappins doubles (15) sont disposés de telle sorte que les positions correspondantes des récipients présentent toujours le même écartement angulaire par rapport aux positions de récipients voisines.

4. Dispositif selon l'une des revendications 1 à 3, **caractérisé en ce que** pour le réglage en hauteur des grappins (15), il est prévu une came de commande (35).

5. Dispositif selon l'une des revendications 1 à 4, **caractérisé en ce qu'**il est prévu des électrodes (24), des conducteurs de micro-ondes (24) et/ou des matériaux de revêtement (24) pour produire un plasma, étant précisé que le réglage en hauteur est de préférence conçu pour que le récipient (22) puisse être déplacé entre des première et seconde positions, l'électrode (24) et/ou le conducteur de micro-ondes (24) et/ou le matériau de revêtement (24) se trouvant à l'extérieur du récipient (22), dans la première position, et au moins en partie à l'intérieur du récipient (22), dans la seconde position.

6. Dispositif selon l'une des revendications 1 à 5, **caractérisé en ce qu'**il est prévu pour le réglage horizontal des grappins (15) une came de commande (34).

7. Dispositif selon l'une des revendications 1 à 6, **caractérisé en ce que** le rotor (25) comprend un corps creux central tournant (25) dont le diamètre représente de préférence au moins 5% ou 10% ou 20% ou 30% ou 40% ou 50% ou 75 ou 80% ou 85% ou 90% du diamètre de la chambre de traitement (2).

8. Dispositif selon la revendication 7, **caractérisé en ce que** le corps creux (25) est relié de manière continue à l'atmosphère.

9. Dispositif selon l'une des revendications 7 ou 8, **caractérisé en ce que** des conduites d'alimentation (28) pour l'intérieur de la chambre de traitement (2) traversent la paroi du corps creux (25), étant précisé qu'il s'agit par exemple de conduites d'amenée ou d'évacuation de gaz ou d'eau de refroidissement ou de lignes haute tension ou haute fréquence.

10. Dispositif selon la revendication 1, **caractérisé en ce que** la zone de traitement par plasma (32) est limitée vers l'extérieur par une paroi de dispositif fixe à l'intérieur de laquelle se trouve un corps creux tournant (25) dont l'intérieur est de préférence destiné à la présence d'une pression atmosphérique et à travers lequel peuvent passer de préférence des conduites d'alimentation (28).

11. Dispositif selon la revendication 1, **caractérisé en ce que** dans la zone du début et de la fin de la zone de traitement par plasma (32), le fond se trouve plus bas que dans une autre partie de ladite zone de traitement de plasma (32).

12. Dispositif selon l'une des revendications 2 à 11, **caractérisé en ce que** les chambres formant sas (3, 4) disposent de grappins (7) pour des récipients (22), qui sont disposés de préférence sur un rotor rotatif.

13. Dispositif selon l'une des revendications 1 à 12, **caractérisé en ce qu'**il est prévu près des chambres formant sas (3, 4) des dispositifs de transfert en étoile (5, 6) grâce auxquels les récipients (22) peuvent être amenés jusqu'aux sas (3) ou éloignés des sas (4).

14. Dispositif selon l'une des revendications 1 à 13, **caractérisé en ce qu'**il est prévu des lampes UV grâce auxquelles les récipients (22) peuvent être éclairés dans la zone du sas (4) avec lequel ils peuvent être évacués de la chambre de traitement (2).

15. Dispositif selon l'une des revendications 1 à 14, **caractérisé en ce qu'**il est prévu des dispositifs pour produire au moins deux plasmas différents.

16. Dispositif selon la revendication 15, **caractérisé en ce qu'**il est prévu au moins deux admissions de gaz pour deux gaz différents.

17. Dispositif selon l'une des revendications 15 ou 16, **caractérisé en ce qu'**il est prévu deux parties différentes pour la production de deux plasmas différents.

18. Dispositif selon l'une des revendications 1 à 17, **caractérisé en ce qu'**il est prévu des lampes UV pour éclairer les récipients (22) dans la zone de la chambre de traitement (2) et/ou dans la zone du transfert des récipients (22) hors de ladite chambre de traitement (2).

19. Dispositif selon les revendications 1 à 18, **caractérisé en ce qu'**un même plasma peut être formé à la fois pour le revêtement et pour la stérilisation.

20. Dispositif selon la revendication 19, **caractérisé en ce qu'**il est conçu pour appliquer comme revêtement Si0₂ et/ou TiO₂, de préférence sous forme d'anatase par modification cristalline, étant précisé que TiO₂ est de préférence intégré dans une matrice SiO₂ sous forme de nanoparticules.
